## Europäisches Patentamt

(19) **European Patent Office**

**Office européen des brevets**

(11) Veröffentlichungsnummer: **0 109 009**
**B1**

(12) # EUROPÄISCHE PATENTSCHRIFT

(45) Veröffentlichungstag der Patentschrift:
**13.05.87**

(51) Int. Cl.⁴: **C 12 P 19/12, A 23 L 1/22**

(21) Anmeldenummer: **83110980.6**

(22) Anmeldetag: **03.11.83**

(54) **Verfahren zur Herstellung von 1-0-alpha-D-Glucopyranosido-D-fructose.**

(30) Priorität: **11.11.82 DE 3241788**

(43) Veröffentlichungstag der Anmeldung:
**23.05.84 Patentblatt 84/21**

(45) Bekanntmachung des Hinweises auf die Patenterteilung:
**13.05.87 Patentblatt 87/20**

(84) Benannte Vertragsstaaten:
**AT BE DE FR GB IT NL SE**

(56) Entgegenhaltungen:
**CHEMICAL ABSTRACTS, Band 97, Nr. 5, 2. August 1982, Seite 459, Nr. 37664y, Columbus, Ohio, USA S.C. ZIESENITZ: "Enzymic preparation and cariogenicity testing of disaccharide alcohols"**

(73) Patentinhaber: **Süddeutsche Zucker-Aktiengesellschaft, Maximilianstrasse 10, D-6800 Mannheim 1 (DE)**

(72) Erfinder: **Munir, Mohammed, Dr., Am Kinderbach 1, D-6719 Kindenheim (DE)**

(74) Vertreter: **Körber, Wolfhart, Dr. et al, Patentanwälte Dipl.-Ing. H. Mitscherlich Dipl.-Ing. K. Gunschmann Dr.rer.nat. W. Körber Dipl.Ing. J. Schmidt-Evers Dipl.-Ing. W. Melzer Steinsdorfstrasse 10, D-8000 München 22 (DE)**

**Beschreibung**

Gegenstand der Erfindung ist die chargenweise oder kontinuierliche Umwandlung von einer wässerigen Lösung von (a) Isomaltulose oder (b) einem Gemisch von Isomaltulose und Saccharose in 1-O-α-D-Glucopyranosido-D-fructose mit Hilfe von freien oder immobilisierten, lebenden oder toten ganzen Zellen bzw. mit dem daraus gewonnenen Enzym in freier oder immobilisierter Form.

1-O-α-D-Glucopyranosido-D-fructose wurde von G. Avigad [Biochem. J. 73, 587 (1959)] durch Einwirkung von Hefen auf ein Gemisch aus Saccharose und Fructose in sehr geringer Ausbeute (0,77% bezogen auf eingesetzte Saccharose) gewonnen. B. M. Lund und G. M. Wyatt [J. Gen. Microbiol. 78 (Pt. 2), 331-6 (1973)] konnten durch die Einwirkung von Erwinia carotovora atroseptica auf 2–4% Saccharose enthaltende Nährlösungen neben 6-O-α-D-Glucopyranosido-D-fructose (Isomaltulose) auch 1-O-α-D-fructose herstellen.

Diese Herstellungsmethoden sind mit dem Nachteil der sehr geringen Ausbeute behaftet, so dass an eine technische Verwertung bisher nicht zu denken war.

Aus der DE-A 3 038 219 ist ein technisches Verfahren zur Herstellung von Isomaltulose (6-O-α-Glucopyranosido-D-fructose) durch enzymatische Umwandlung aus Saccharose mit Hilfe von immobilisierten Bakterienzellen bekanntgeworden; im Zuge dieses Verfahrens wird in Abhängigkeit von den Bedingungen die 1-O-α-D-Glucopyranosido-D-fructose als Nebenprodukt gebildet.

Demgegenüber wurde nun überraschenderweise gefunden, dass man durch die Einhaltung geeigneter Reaktionsbedingungen aus sowohl einer wässerigen Lösung von Isomaltulose als auch einer Isomaltulose und Saccharose enthaltenden Lösung mit Hilfe von Isomaltulose aus Saccharose bildenden Mikroorganismen eine Produktlösung herstellen kann, die als Hauptkomponente die 1-O-α-D-Glucopyranosido-D-fructose enthält. Dabei ist es gleichgültig, ob die Mikroorganismen als ganze Zellen in lebender oder toter, in freier oder immobilisierter Form eingesetzt werden, oder ob man zunächst aus den Zellen das Enzym gewinnt und dieses dann als freies Enzym oder in immobilisierter Form zur Anwendung bringt.

Das Verfahren zur Herstellung von 1-O-α-D-Glucopyranosido-D-fructose durch enzymatische Umwandlung ist erfindungsgemäss dadurch gekennzeichnet, dass man eine wässerige Lösung von (a) Isomaltulose oder (b) einem Gemisch von Isomaltulose und Saccharose mit freien oder immobilisierten, lebenden oder toten ganzen Zellen oder mit dem freien oder immobilisierten Enzymextrakt von Isomaltulose aus Saccharose bildenden Mikroorganismen in Kontakt bringt, aus der so behandelten Lösung durch chromatographische Trennung an Ionenaustauschern oder anderen geeigneten Trennmaterialien die 1-O-α-D-Glucopyranosido-D-fructose zunächst als wässerige Lösung gewinnt und dann durch an sich bekannte Methoden in trockene Form überführt.

Zweckmässige Ausgestaltungen des Verfahrens sind in den Unteransprüchen gekennzeichnet.

Die 1-O-α-D-Glucopyranosido-D-fructose ist als Süssungsmittel aus Chemical Abstracts, Band 97, Nr. 5, 2. August 1982, Seite 459, Referat Nr. 37664y, bekannt geworden; sie kann zu diesem Zweck in Nahrungs-, Genuss- und Futtermitteln in fester oder flüssiger Form und/oder Mischung mit Süssstoffen mit gegenüber Saccharose gleicher oder höherer Süsskraft bzw. in Mischung mit Fructose, Sorbit, Xylit, Palatinit® oder anderen Disaccharidalkoholen mit der Saccharose vergleichbarer Süsskraft oder mit Isomaltulose mit der Saccharose vergleichbarer Löslichkeit eingesetzt werden. Einsatzgebiete sind etwa Karamellen, Speiseeis oder Marmelade.

In diesem Zusammenhang wird noch bemerkt, dass nach vergleichenden Geschmackstests die Süsskraft von 1-O-α-Glucopyranosido-D-fructose 45% der der Saccharose beträgt und gleich der Süsskraft der Isomaltulose ist.

Die Löslichkeit von 1-O-α-Glucopyranosido-D-fructose in Wasser ist im Vergleich zur Saccharose oder Isomaltulose besonders hoch. So lösen sich bei 20°C in 1 g Wasser 2 g Saccharose, 0,6 g Isomaltulose aber über 4 g 1-O-α-D-Glucopyranosido-D-fructose. Somit kann man zum Beispiel bei der Verwendung von Isomaltulose Nachteile, die durch deren geringere Löslichkeit entstehen, durch den Einsatz von Mischungen mit 1-O-α-Glucopyranosido-D-fructose kompensieren. Erste Untersuchungen scheinen zu erhärten, dass aufgrund der geringen Säurebildung nach Inkubation mit Streptococcus mutans dieser Zucker als nicht kariogen einzustufen ist. Ausserdem scheint er auch keinen Einfluss auf die Plaquepolysaccharid-Bildung zu haben. Darüber hinaus wird 1-O-α-D-Glucopyranosido-D-fructose von den Enzymsystemen des Dünndarmes des Menschen nur schwer gespalten und daher nur teilweise und verzögert resorbiert.

Das anmeldungsgemässe Verfahren wird anhand von folgenden Beispielen näher erläutert.

Beispiel 1

a) Von einer Abimpfung des Stammes Protaminobacter rubrum (CBS 574.77) werden Zellen von 10 ml eines sterilen Nährsubstrates, bestehend aus 8 kg Dicksaft aus einer Zuckerfabrik (Trockensubstanzgehalt = 65%), 2 kg Maisquellwasser, 0,1 kg $(NH_4)_2HPO_4$ und 89,9 kg dest. Wasser (bei Bedarf auf pH 7,2 eingestellt) abgeschwemmt. Diese Suspension dient als Impfgut für die Schüttelmaschinen-Vorkultur in 1-l-Kolben mit 200 ml Nährlösung obiger Zusammensetzung.

Nach einer 30stündigen Bebrütungszeit bei 29°C werden mit je 20 Kolben (Gesamtinhalt 4 l) 16 l Nährlösung obiger Zusammensetzung in einem 30-l-Kleinfermenter beimpft und bei 29°C mit 20 l Luft pro Minute und einer Rührgeschwindigkeit von 350 UpM fermentiert. Die anwachsende Keimzahl wird mikroskopisch bestimmt. Nach Erreichen von Keimzahlen über $5 \times 10^9$ Keimen/ml wird die Fermentation durch Abstellen der Rührung und Belüftung und Absenken der Temperatur auf 20°C abgebrochen. Der Fermenter-Inhalt verbleibt unter sterilen Bedingungen im Fermenter und dient als Enzym-Quelle für die nachfolgenden Beispiele.

b) Gegen Ende der Fermentation in Beispiel 1 a) wird folgende Zusammensetzung des Trockensubstanzgehaltes des Substrates festgestellt:

| | |
|---|---|
| Fructose | 2– 4 Gew.% |
| Glucose | 0,5– 1 Gew.% |
| Saccharose | 5–10 Gew.% |
| Isomaltulose | 75–85 Gew.% |
| 1-O-α-D-Glucopyranosido-D-fructose | 5–10 Gew.% |
| Oligomere | 0,5– 2 Gew.% |

Wird diese Fermenterbrühe 4 Stunden bei 30°C unter langsamem Rühren, jedoch ohne Belüftung, weiterbebrütet, dann ergibt sich die folgende Zusammensetzung:

| | |
|---|---|
| Fructose | 5– 8 Gew.% a. TS-Gehalt |
| Glucose | 2– 5 Gew.% a. TS-Gehalt |
| Saccharose | 0–0,5 Gew.% a. TS-Gehalt |
| Isomaltulose | 65– 72 Gew.% a. TS-Gehalt |
| 1-O-α-D-Glucopyrano-sido-D-fructose | 10–20 Gew.% a. TS-Gehalt |
| Oligomere | 3– 6 Gew.% a. TS-Gehalt |

c) Die Nährlösung nach Beispiel 1 b) wird durch Zentrifugieren von den Zellen befreit, auf einen TS-Gehalt von 80% eingedampft und unter Zugabe von Isomaltulose-Impfkristallen in einen Kühlungskristallisator mit einer Kühlrate von 1–5°C/h auf 20°C gekühlt. Die entstandenen Isomaltulose-Kristalle werden an einer Siebkorbzentrifuge entfernt. Die Mutterlauge wird erneut auf 80% TS-Gehalt eingedampft und unter Zugabe von Isomaltulose-Impfkristallen in einen Kühlungskristallisator mit einer Kühlrate von 0,5–2°C/h auf 20°C abgekühlt. Die entstandenen Isomaltulose-Kristalle werden an einer Siebkorbzentrifuge abgetrennt. Diese zweite Mutterlauge wird auf einen TS-Gehalt von 85% eingedampft und mit Isomaltulose-Kristallen beimpft. Diesen Sirup verdünnt man etwa 1:1 volumenmässig mit Methanol, Ethanol oder einem anderen Alkohol und kühlt die alkoholische Lösung auf 4°C ab. Die auskristallisierte Isomaltulose wird durch Zentrifugieren entfernt. Diese dritte Mutterlauge weist folgende ungefähre Zusammensetzung des TS-Gehaltes auf:

| | |
|---|---|
| Fructose | 8–12 Gew.% |
| Glucose | 4– 6 Gew.% |
| Saccharose | 0– 2 Gew.% |
| Isomaltulose | 45–50 Gew.% |
| 1-O-α-Glucopyrano-sido-D-fructose | 30–35 Gew.% |
| Oligomere | 3– 6 Gew.% |

d) Die nach Beispiel 1 c) angefallene Mutterlauge wird durch Verdampfung von Methanol befreit, auf 15–20% TS-Gehalt eingestellt und bei 37°C mit Bäckerhefe vergoren bis in der Lösung keine Saccharose, Glucose und Fructose mehr nachweisbar sind. Die Hefe wird absepariert. Die klare Lösung wird auf 50% TS-Gehalt eingedampft und dann über eine chromatographische Trennsäule getrennt. Die 1-O-α-Glucopyranosido-D-fructose enthaltende Fraktion wird gesammelt und daraus nach Vollentsalzung die 1-O-α-Glucopyranosido-D-fructose durch Kristallisation, Gefriertrocknung, Sprühtrocknung oder andere ähnliche Verfahren in trockener Form gewonnen.

e) Die in Beispiel 1 d) aufgeführte Vergärung der Mutterlauge mit Hefe ist nicht zwingend notwendig. Man kann auch darauf verzichten und die Mutterlauge direkt einer chromatographischen Trennung unterwerfen. In diesem Fall wird die nach Beispiel 1 c) angefallene Mutterlauge durch Verdampfung von Methanol befreit, auf 50% TS-Gehalt eingestellt und dann über eine chromatographische Trennsäule getrennt. Aus der 1-O-α-Glucopyranosido-D-fructose enthaltenden Fraktion wird nach Vollentsalzung die 1-O-α-Glucopyranosido-D-fructose durch Kristallisation, Gefriertrocknung, Sprühtrocknung oder andere ähnliche Verfahren in trockener Form gewonnen.

Ausbeute: 14,3 kg pro 100 kg eingesetzte Saccharose.

Die bei der chromatographischen Trennung angefallenen anderen Zucker, wie Fructose, Glucose und Isomaltulose, stellen keinen Verlust dar, sie können geeigneten Verwendungszwecken (zum Beispiel Isomaltulose zur Palatinit®-Herstellung) zugeführt werden.

Beispiel 2

Immobilisierte Zellen von Protaminobacter rubrum (CBS 574.77), hergestellt nach Beispiel 1 a) und einem der an sich bekannten Methoden der Zellimmobilisierung, werden in einen temperierbaren Säulenreaktor gefüllt und kontinuierlich mit einer Isomaltuloselösung (30% TS-Gehalt) bei 50°C durchströmt. Die Isomaltuloselösung wird dabei im Kreislauf geführt. Die Umsetzung wird so lange durchgeführt, bis im Produktstrom keine Isomaltulose mehr vorhanden ist. Die Gesamtverweilzeit beträgt dabei ca. 150 Stunden. Danach hat die Produktlösung folgende beispielhafte Zusammensetzung:

| | |
|---|---|
| Fructose | 31% a. TS-Gehalt |
| Glucose | 31% a. TS-Gehalt |
| 1-O-α-Glucopyranosido-D-fructose | 38% a. TS-Gehalt |

Diese Produktlösung wird auf 50% TS-Gehalt eingedampft und dann über eine chromatographische Trennsäule getrennt. Aus der 1-O-α-Glucopyranosido-D-fructose enthaltenden Fraktion wird dieser Zucker durch Kristallisation, Gefriertrocknung, Sprühtrocknung oder anderen ähnlichen Verfahren in trockener Form gewonnen.

Ausbeute: ca. 38% der eingesetzten Isomaltulose.

## Patentansprüche

1. Verfahren zur Herstellung von 1-O-α-D-Glucopyranosido-D-fructose durch enzymatische Umwandlung aus Isomaltulose, dadurch gekennzeichnet, dass man eine wässerige Lösung von (a) Isomaltulose oder (b) einem Gemisch von Isomaltulose und Saccharose mit freien oder immobilisierten, lebenden oder toten ganzen Zellen oder mit dem freien oder immobilisierten Enzymextrakt von Isomaltulose aus Saccharose bildenden Mikroorganismen in Kontakt bringt, aus der so behandelten Lösung durch chromatographische Trennung an Ionenaustauschern oder anderen geeigneten Trennmaterialien die 1-O-α-D-Glucopyranosido-D-fructose zunächst als wässerige Lösung gewinnt und dann durch an sich bekannte Methoden in trockene Form überführt.

2. Verfahren nach Anspruch 1, dadurch gekennzeichnet, dass man vor der chromatographischen Trennung aus der Mutterlauge die mit Hefe vergärbaren Zucker mit freier oder immobilisierter Hefe vergärt.

3. Verfahren nach Anspruch 1, dadurch gekennzeichnet, dass man aus der behandelten Lösung einen Teil der Isomaltulose durch Kristallisation abtrennt und mit der Mutterlauge die chromatographische Trennung durchführt.

4. Verfahren nach Anspruch 1, dadurch gekennzeichnet, dass die Lösungen in Konzentrationen von 20 Gew.% bis 55 Gew.%, kontinuierlich oder chargenweise, mit den Zellen oder dem Enzymextrakt aus Isomaltulose aus Saccharose bildenden Mikroorganismen behandelt werden.

5. Verfahren nach Anspruch 1, dadurch gekennzeichnet, dass die Lösungen mit den Zellen oder dem Enzymextrakt bei 25°–55°C, vorzugsweise 30°–55°C, behandelt werden.

6. Verfahren nach Anspruch 1, dadurch gekennzeichnet, dass es sich bei den verwendeten Mikroorganismen um Protaminobacter rubrum (CBS 574.77), Serratia plymuthica (ATCC 15 928), Serratia marcescens (NCIB 8285), Leuconostoc mesenteroides [NRRL-B-512 F (ATCC 1083a)] und Erwinia rhapontici (NCPPB 1578), vorzugsweise um Protaminobacter rubrum (CBS 574.77), handelt.

## Claims

1. A process for preparing 1-O-α-D-glucopyranosido-D-fructose by the enzymatic conversion of isomaltulose, characterized in that an aqueous solution of a) isomaltulose or b) a mix of isomaltulose and sucrose are contacted with free or immobilized, living or dead complete cells or with the free or immobilized enzyme extract of microorganisms which form isomaltulose from sucrose, that 1-O-α-D-glucopyranosido-D-fructose is obtained first as an aqueous solution from the solution thus treated by chromatographic separation at ion exchangers or other suitable separating materials and that it is then transferred into dry form by methods known per se.

2. The process according to claim 1, characterized in that prior to the chromatographic separation the yeast-fermenting sugars of the mother liquor are subjected to fermentation with free or immobilized yeast.

3. The process according to claim 1, characterized in that the treated solution is subjected to crystallization to remove a portion of isomaltulose and that chromatographic separation is carried out with the mother liquor.

4. The process according to claim 1, characterized in that the solutions having a concentration of 20 to 55% by weight are treated continuously or in batches with the cells or the enzyme extract of microorganisms which form isomaltulose from sucrose.

5. The process according to claim 1, characterized in that the solutions are treated with the cells or the enzyme extract at a temperature of 25°C–55°C, preferrably at 30°C–55°C.

6. The process according to claim 1, characterized in that the microorganisms are selected from the group consisting of Protaminobacter rubrum (CBS 574.77), Serratia plymuthica (ATCC 15 928), Serratia marcescens (NCIB 8285), Leuconostoc mesenteroides [NRRL-B-512 F (ATCC 1083a)] and Erwinia rhapontici (NCPPB 1578).

## Revendications

1. Procédé pour la préparation de 1-O-alpha-D-glucopyranosido-D-fructose par transformation enzymatique à partir d'isomaltulose, caractérisé en ce qu'on met en contact une solution aqueuse (a) d'isomaltulose ou (b) d'un mélange d'isomaltulose et de saccharose avec des cellules entières, libres ou immobilisées, vivantes ou mortes ou avec l'extrait d'enzyme libre ou immobilisé d'isomaltulose provenant de microorganismes générant de la saccharose, en ce qu'à partir de la solution ainsi traitée on obtient d'abord sous forme de solution aqueuse le 1-O-alpha-D-glucopyranosido-D-fructose, par séparation chromatographique sur des échangeurs d'ions ou sur d'autres substances de séparation appropriées, puis en ce qu'on le sèche par des méthodes connues.

2. Procédé suivant la revendication 1, caractérisé en ce qu'avant la séparation chromatographique à partir de la liqueur mère, on fait fermenter le sucre – fermentable avec de la levure – avec de la levure libre ou immobilisée.

3. Procédé suivant la revendication 1, caractérisé en ce qu'à partir de la solution traitée, on sépare une partie de l'isomaltulose par cristallisation et qu'on réalise avec la liqueur mère la séparation chromatographique.

4. Procédé suivant la revendication 1, caractérisé en ce que les solutions sont traitées d'une manière continue ou fractionnée, à des concentrations de 20 à 55% en poids, avec les cellules ou l'extrait d'enzyme d'isomaltulose provenant de microorganismes générant de la saccharose.

5. Procédé suivant la revendication 1, caractérisé en ce que les solutions sont traitées à 25–55°C et, de préférence, à 35–55°C avec les cellules ou l'extrait d'enzyme.

6. Procédé suivant la revendication 1, caractérisé en ce que les microorganismes employés sont le protaminobacter rubrum (CBS 574.77), la serratia plymuthica (ATCC 15 928), la serratia marcescens (NCIB 8 285), le leuconostoc mesenteroides [NRRL-B-512 F (ATCC 1 083a)] et l'erwinia rhapontici (NCPPB 1 578), mais de préférence le protaminobacter rubrum (CBS 574.77).